# EUROPEAN PATENT APPLICATION

(11) **EP 4 356 948 A1**
(43) Date of publication of application: **24.04.2024**
(21) Application number: 22201954.9
(22) Date of filing: 17.10.2022
(51) Int. Cl.: A61M 25/01, A61M 25/00, A61M 25/06

(54) **A RAPID EXCHANGE GUIDE CATHETER FOR GUIDING A GUIDEWIRE THROUGH CORONARY ARTERIES**

(71) Applicant: IMDS R&D BV, 9301 NZ Roden (NL)
(72) Inventor: SCHULTING, Edwin Alexander, 9301 NZ Roden (NL)
(74) Representative: V.O.

(57) **Abstract**

A rapid exchange guide catheter (1) according to the invention comprises a flexible bent-away tubular segment (15) having a pre-formed shape corresponding to a one-direction blunt bend line (7) with an overall deflection angle (18) value in a range between 30 and 90 degrees and with shape retention memory. The catheter has a distal guide port (14) at the distal catheter end (4) as a first optional exit for a guidewire, and a side guide port (19) as a second optional exit for the guidewire. The side guide port (19) is situated on a side of the catheter's tubular element (11) facing diametrically away from the sideward deflection direction (17) of the one-direction blunt bend line. According to the invention, guiding and delivering a guidewire via a bifurcation of the coronary arteries can be performed easier and quicker.

## Description

The invention relates to guiding a guidewire through coronary arteries and for delivering said guidewire into the coronary arteries. More particularly, the invention relates to guiding and delivering a guidewire via at least one bifurcation of the coronary arteries.

Generally, such kind of "wiring" coronary arteries is difficult, sometimes even more or less impossible.

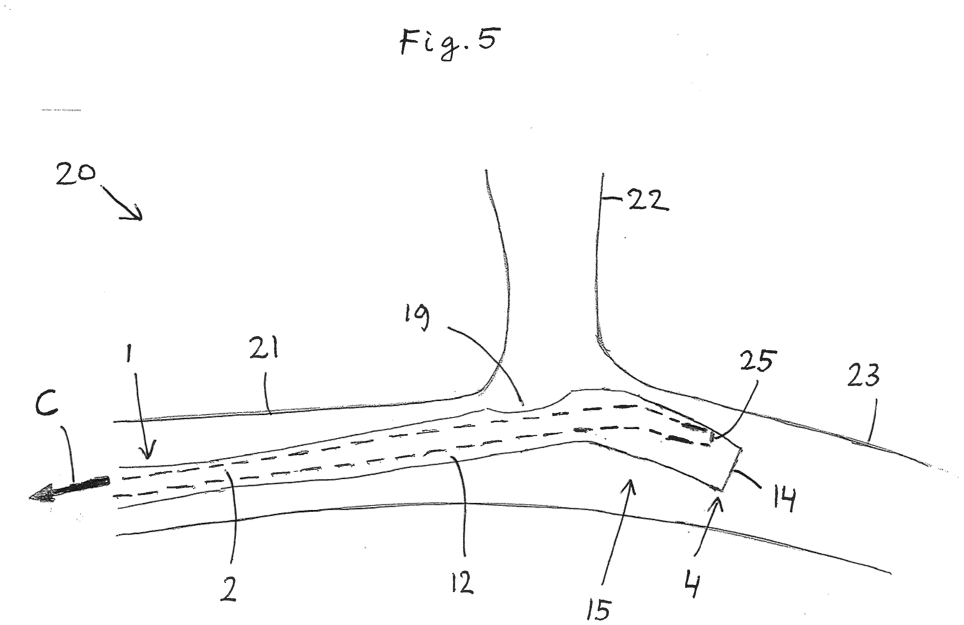

US 7,115,134 B2, US 7,867,219 B2 and US 9,168,353 B2 disclose examples of the use of a guide catheter for steering a guidewire via bifurcations of the coronary arteries, wherein the guide catheter has a flexible distal tip having a pre-formed angle of curvature with shape retention. Figs. 5A-5F of said references US 7,115,134 B2, US 7,867,219 B2 and US 9,168,353 B2 show how the catheter and the guidewire are used together to guide the catheter to a desired location in a body passage of a coronary arterial tree. Generally, this involves introducing the guidewire followed by the catheter and advancing the catheter and guidewire concentrically until reaching a targeted branched passageway of a bifurcation. The guidewire can then be withdrawn into the catheter allowing the catheter's pre-formed distal tip to resume its pre-formed angle of curvature. Said references US 7,115,134 B2, US 7,867,219 B2 and US 9,168,353 B2 teach that, by rotating the catheter outside the patient-body at the proximal end of the catheter, the pre-formed distal tip can be rotated and directed to the origin of the targeted branched passageway. The guidewire can then be advanced and using the plurality of forces the desired angle of curvature of the pre-formed distal tip of the catheter can be created, thereby allowing the guidewire to be advanced into the targeted branched passageway.

However, the devices and methods known from said references US 7,115,134 B2, US 7,867,219 B2 and US 9,168,353 B2 have a drawback which is explained as follows. In practice it often happens that catheters have a preferred orientation in a coronary arterial tree. This is due to interactions between the configurations of the catheter and the coronary arterial tree. As a result thereof it is sometimes very difficult or impossible for the medical practitioner to torque the catheter from outside the patient-body at the proximal end of the catheter in such manner that the pre-formed distal tip of the catheter is rotated and directed to the origin of the targeted branched passageway of the bifurcation. The reason is that the preferred orientation of the catheter relative to a certain blood vessel structure in combination with the known phenomenon called "whip" in many cases causes that torquing of the catheter practically all the time results into one or more exact 360 degrees rotations of the distal tip. This way the distal tip practically always ends up into its erroneous starting orientation, which is not directed to the origin of the targeted branched passageway at the bifurcation. In such cases said whip phenomenon is usually caused by the torquing of the catheter from outside the patient-body, which is far away from the distal tip. As torque builds during torquing, energy is stored in the catheter. And when the catheter ultimately and suddenly rotates, such rotation is caused by the fact that a certain uncontrollable amount of the stored energy is suddenly released. In fact, the catheter does not use all the stored energy to automatically arrive at its preferred orientation relative to the blood vessel structure again. Instead, the remaining unused part of the stored energy is not enough to overcome the resistance from the stable support of the catheter within the blood vessel structure in the catheter's preferred orientation, in which the catheter's distal tip is not directed to the origin of the targeted branched passageway. Clearly, this is a frustrating experience for medical practitioners.

It is an object of the present invention to provide at least an alternative solution for wiring coronary arteries, and more particularly to provide a solution according to which guiding and delivering a guidewire via a bifurcation of the coronary arteries can be performed easier and quicker.

For that purpose the invention provides a rapid exchange guide catheter according to the appended independent claim 1. Preferable embodiments of the invention are provided by the appended dependent claims 2-6.

Accordingly, the invention provides a rapid exchange guide catheter configured for use through coronary arteries for guiding a guidewire through said coronary arteries and for delivering said guidewire into said coronary arteries,
wherein the catheter has a proximal catheter end, a distal catheter end and a longitudinal direction extending from the proximal catheter end to the distal catheter end, wherein the catheter comprises a proximal catheter segment and a distal catheter segment, which are mutually interconnected in said longitudinal direction, the proximal catheter segment ending at the proximal catheter end, the distal catheter segment ending at the distal catheter end, the distal catheter segment being a rapid exchange segment of the catheter and comprising a tubular element ending at the distal catheter end, wherein the tubular element comprises one and only one guide lumen, a proximal guide port at a proximal guide lumen end and a distal guide port at a distal guide lumen end being the distal catheter end, wherein the proximal guide port, the guide lumen and the distal guide port are each configured for guiding a guidewire therethrough,
wherein the tubular element comprises a bent-away tubular segment ending at the distal catheter end and a straight tubular segment immediately proximal from the bent-away tubular segment, the bent-away tubular segment and the straight tubular segment being flexible, and the bent-away tubular segment and the straight tubular segment having pre-formed shapes, respectively, with shape retention memories to return to pre-formed states corresponding to said pre-formed shapes, respectively,
wherein, as seen in said pre-formed states of the straight tubular segment and the bent-away tubular segment, the pre-formed shape of the straight tubular segment corresponds to the straight tubular segment having a pre-formed straight center line in said longitudinal direction of the catheter, and the pre-formed shape of the bent-away tubular segment corresponds to the bent-away tubular segment having a pre-formed bent center line in said longitudinal direction of the catheter, said pre-formed bent center line forming a one-direction blunt bend line, which in a sideward deflection direction relative to said pre-formed straight center line makes an overall deflection angle having a value in a range between 30 and 90 degrees relative to said pre-formed straight center line,
wherein the bent-away tubular segment has a flexibility that allows the pre-formed bent center line to be at least straightened by advancing a guidewire through the bent-away tubular segment,
wherein the tubular element further comprises a side guide port, said side guide port being configured for guiding a guidewire therethrough,
and wherein the side guide port, as seen in said pre-formed states of the straight tubular segment and the bent-away tubular segment, and as seen in a circumferential direction around said longitudinal direction of the catheter, is situated on a side of the tubular element facing diametrically away from said sideward deflection direction of said one-direction blunt bend line,
so that the distal guide port and the side guide port form two selectively choosable alternative exits through which a distal guidewire end of a guidewire can be guided out of the guide lumen, when the guidewire is advanced distally along said longitudinal direction of the catheter.

Briefly summarized the invention has the following combined key features:
- the guide catheter is a rapid exchange catheter, wherein the distal catheter segment of the catheter comprises the tubular element;
- the flexible bent-away tubular segment of the catheter has the pre-formed shape corresponding to the one-direction blunt bend line with deflection angle value in a range between 30 and 90 degrees and with shape retention memory;
- the catheter has the distal guide port at the distal catheter end as a first optional exit for the guidewire; and
- the catheter has the side guide port as a second optional exit for the guidewire, the side guide port facing diametrically away from the sideward deflection direction of the one-direction blunt bend line of the bent-away tubular segment.

The favourable effects of these combined key features are briefly described in connection with the following example use of the catheter according to the invention.

The rapid exchange guide catheter according to the invention is navigated through coronary arteries in a first condition in which a guidewire has been inserted into the distal guide lumen of the catheter to such an extent that the distal guidewire end of the guidewire has been advanced distally into the bent-away tubular segment and that the pre-formed bent center line of the bent-away tubular segment has been deformed a little bit towards a straightened condition thereof. In the present example use it is assumed that in said first condition of the catheter and guidewire, the distal guidewire end is distal from the side guide port. In the present example it is further assumed that in preparing said first condition, the medical practitioner had deformed the guidewire tip a little bit towards following the pre-formed shape of the bent-away tubular segment a little bit. Needless to say, at all times during the navigation process the proximal catheter end and the proximal guidewire end are of course outside the patient-body so that the medical practitioner is able to manipulate these proximal ends during the navigation process.

When, in said first condition, the distal catheter end has reached a first bifurcation of the coronary arteries, the guidewire is retracted a little bit relative to the catheter in proximal direction, to such an extent that the bent-away tubular segment is allowed to at least partly return a bit further towards its pre-formed shape. This way a second condition of the catheter and guidewire has been obtained. In this second condition, the distal guidewire end is still inside the guide lumen, and in the present example we assume that it is still distal from the side guide port.

Thanks to the fact that in the second condition the bent-away tubular segment is allowed to at least partly return towards its pre-formed shape, and thanks to the specified one-direction blunt bend line character of the pre-formed shape, there is a very good chance (a more or less fifty-fifty chance) that the bent-away tubular segment deforms to partly extend into the targeted branched passageway of the first bifurcation. A little bit of moving the guidewire to-and-fro relative to the catheter, which can be done axially and/or rotationally, may help to redirect the bent-away tubular segment a bit so that it will extend better into the targeted branched passageway.

Next, in this lucky scenario ("plan-A" scenario) the guidewire may simply be advanced distally out of the distal guide port, so that the targeted branched passageway of the first bifurcation can be wired right away. If after the first bifurcation a second bifurcation is encountered in the coronary arteries, the above-described process relating to the first bifurcation can then be repeated for a new targeted branched passageway of the second bifurcation.

In absence of the above-mentioned lucky plan-A scenario at the first bifurcation, the present invention further allows for a "plan-B" procedure, which is now explained as follows.

Hence it is now assumed that also in the plan-B scenario the catheter and guidewire have attained the above-mentioned first and second conditions. However, this time the bent-away tubular segment in the second condition of the plan-B scenario is extending into the other, non-targeted branched passageway of the first bifurcation. And this time the slight retraction of the guidewire relative to the catheter in proximal direction has led to the situation that the bent-away tubular segment, which has at least partly returned a bit further towards its pre-formed shape, in the second condition of the plan-B scenario is pushing on a blood vessel wall of the non-targeted branched passageway. This pushing provides the catheter tip with some extra stability within the blood vessel structure, which is an advantage of the invention since it is helpful for the further plan-B procedure. Starting-off from the second condition of the plan-B scenario, the guidewire is retracted yet a bit further relative to the catheter in proximal direction in such manner that the distal guidewire end of the guidewire comes within the longitudinal range of the side guide port. Furthermore, the guidewire is rotated relative to the catheter over approximately 180 degrees about the longitudinal direction. This way the catheter and guidewire attain a third condition of the plan-B scenario in which the distal guidewire end is projecting a bit out of the side guide port.

Starting-off from this third condition the guidewire may simply be further advanced distally out of the side guide port. There is a very good chance that the guidewire will thus be entered right away into the targeted branched passageway of the first bifurcation, so that the targeted branched passageway of the first bifurcation can be wired. A little bit of rotating the guidewire to-and-fro about the longitudinal direction may help to redirect the guidewire into the targeted branched passageway.

In this plan-B scenario, for the further wiring after the first bifurcation, the rapid exchange guide catheter can be first removed out of the patient-body, while retaining the guidewire into the patient-body. Next, the rapid exchange guide catheter can be re-inserted into the patient-body and over the guidewire that already entered the targeted branched passageway of the first bifurcation. Alternatively, the rapid exchange guide catheter can be replaced by another rapid exchange guide catheter. Thanks to the rapid exchange character of the guide catheter, such a removal and re-insertion/replacement of the catheter in a plan-B scenario can be done easily and quickly.

Accordingly, it has now been made clear that the invention allows to easily and quickly direct a guidewire into a targeted branched passageway of a bifurcation.

In a preferable embodiment of a rapid exchange guide catheter according to the invention, said value, relative to said pre-formed straight center line, of said overall deflection angle of said one-direction blunt bend line of said pre-formed bent center line of said bent-away tubular segment is in a range between 40 and 85 degrees, more preferably between 50 and 80 degrees, yet more preferably between 60 and 75 degrees, yet more preferably between 68 and 71 degrees, and most preferably is equal to 70 degrees.

In another preferable embodiment of a rapid exchange guide catheter according to the invention, at least part of said side guide port is located in the straight tubular segment.

More preferably, said side guide port as a whole is located in the straight tubular segment.

In another preferable embodiment of a rapid exchange guide catheter according to the invention, said side guide port is elongate in said longitudinal direction of the catheter. This improves guiding a guidewire with its distal guidewire end through said side guide port.

In another preferable embodiment of a rapid exchange guide catheter according to the invention said pre-formed bent center line further forms a straight line immediately distal from said one-direction blunt bend line and distally ending at the distal catheter end, as seen in said pre-formed states of the straight tubular segment and the bent-away tubular segment.

In the following, the invention is further elucidated with reference to nonlimiting embodiments and with reference to the schematic figures in the appended drawing, in which the following is shown.

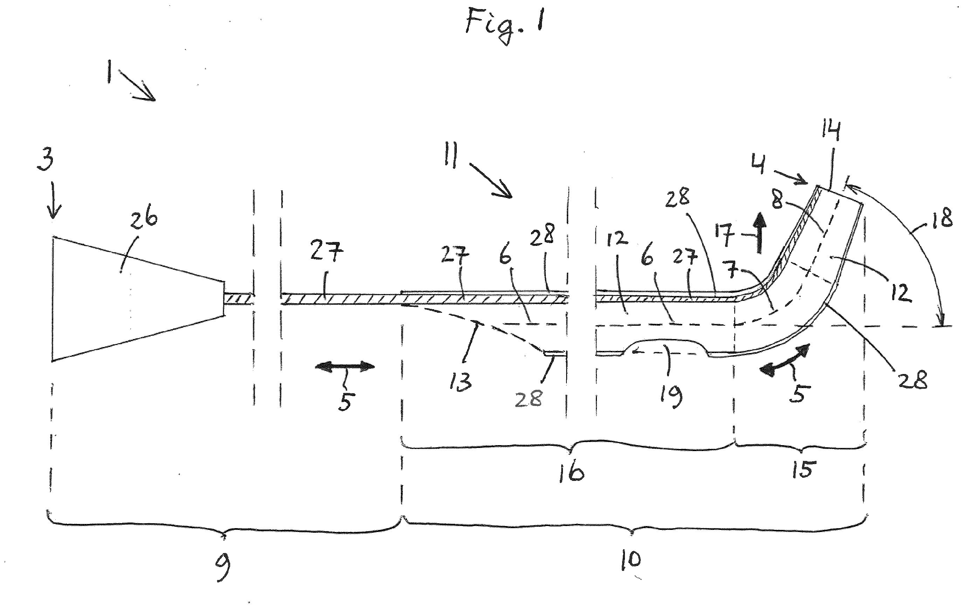

Fig. 1 shows, in longitudinal sectional view, an example of an embodiment of a rapid exchange guide catheter according to the invention, wherein the bent-away tubular segment and the straight tubular segment of the catheter are in their pre-formed states, respectively.

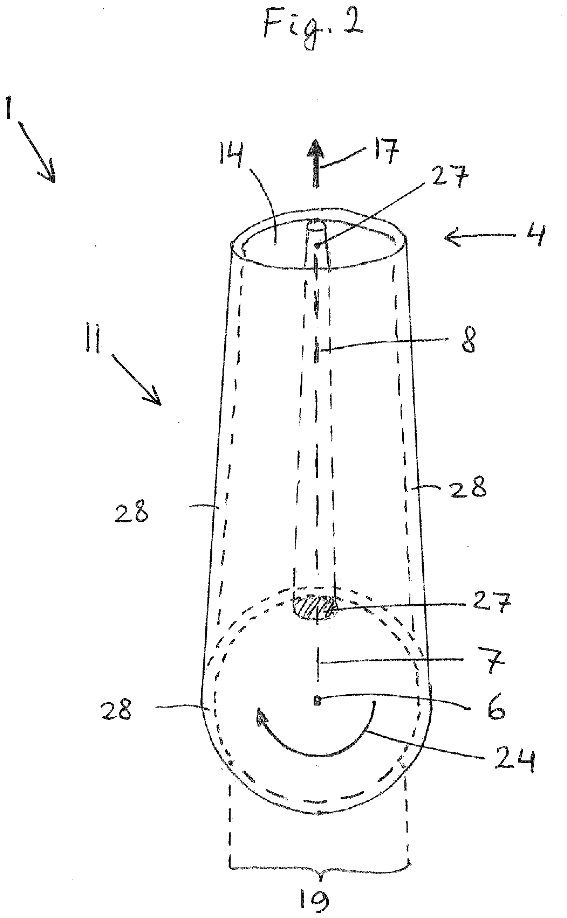

Fig. 2 shows an end view (partly transparent) of the catheter in the condition of Fig. 1, wherein the end view is taken in proximal direction parallel to the pre-formed straight center line of the straight tubular segment of the catheter.

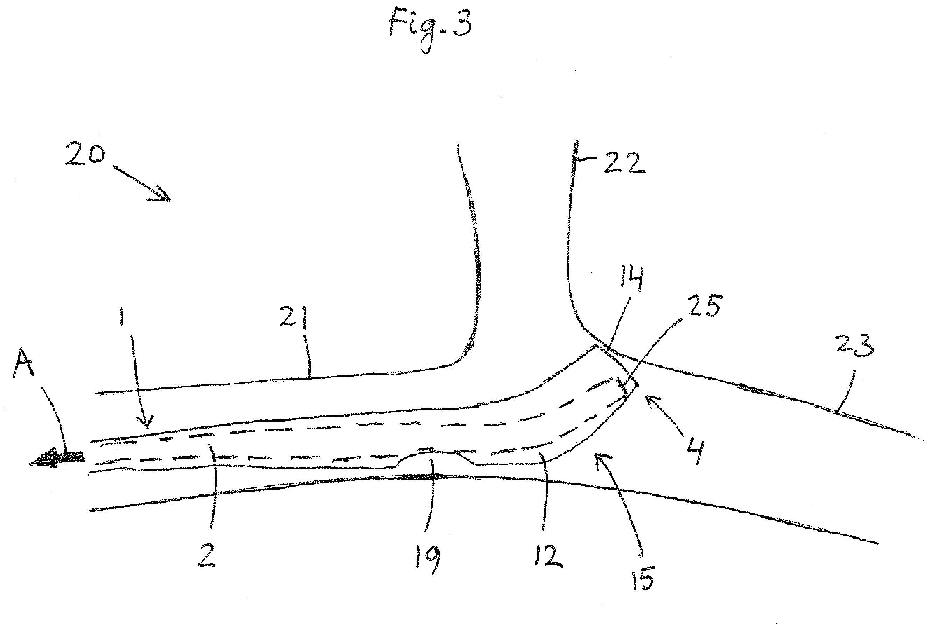

Fig. 3 shows, in longitudinal sectional view, a part of the catheter of Fig. 1 in a first condition in which the bent-away tubular segment of the catheter during navigation of the catheter has reached a bifurcation of the coronary arteries, while a guidewire is extending through the guide lumen of the rapid exchange segment of the catheter.

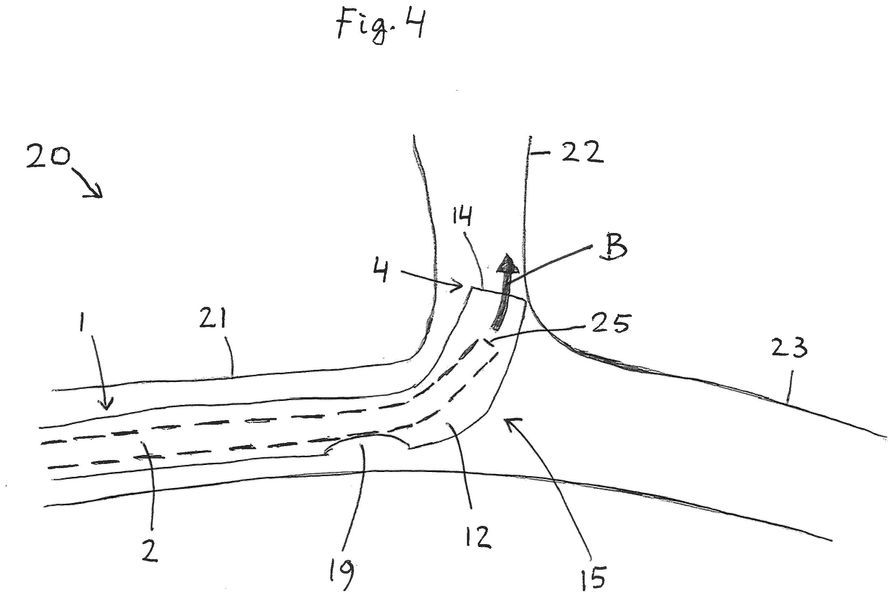

Fig. 4 shows the situation of Fig. 3 again, however wherein this time the guidewire has been retracted a little bit relative to the catheter in proximal direction, whereby a second condition of the catheter and guidewire has been obtained in which the bent-away tubular segment of the catheter partly extends into a targeted branched passageway of the first bifurcation.
Fig. 5 shows the situation of Fig. 3 again, however wherein this time the tubular element of the distal catheter segment has reached the bifurcation of the coronary arteries in a different orientation relative to the two branches of the bifurcation.

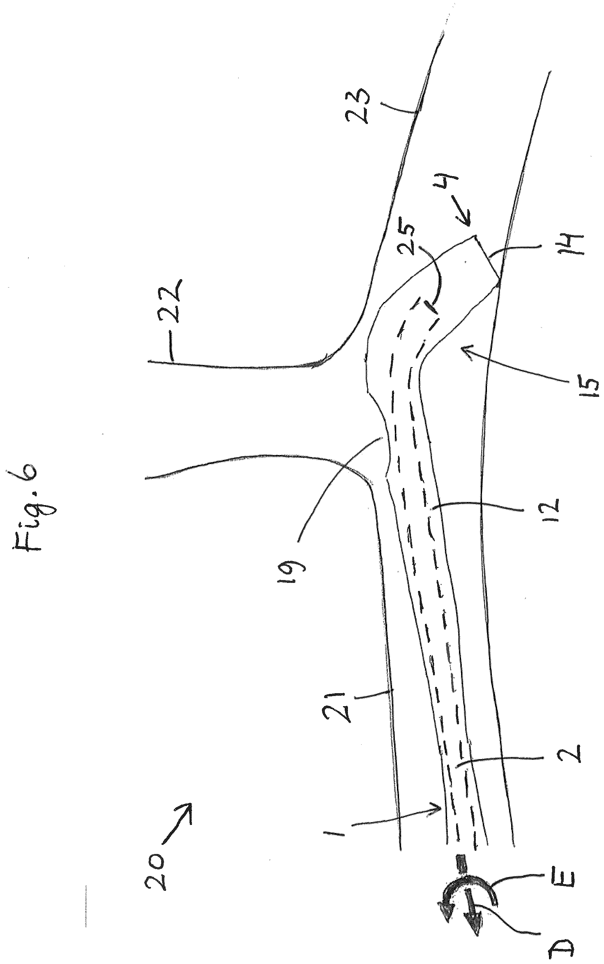

Fig. 6 shows the situation of Fig. 5 again, however wherein this time the guidewire has been retracted a little bit relative to the catheter in proximal direction, whereby a second condition of the catheter and guidewire has been obtained similar to the situation of Fig. 4, however wherein this time the bent-away tubular segment of the catheter partly extends into the NON-targeted branched passageway of the first bifurcation.

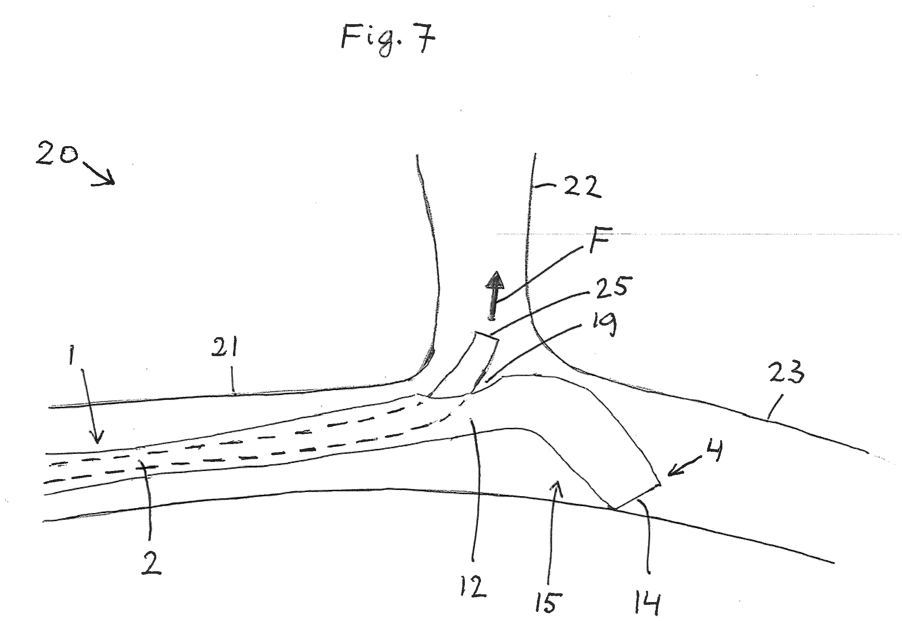

Fig. 7 shows the situation of Fig. 6 again, however wherein this time the guidewire has been retracted relative to the catheter yet a little bit further in proximal direction, and has further been rotated relative to the catheter over approximately 180 degrees about the catheter's longitudinal direction, whereby a third condition of the catheter and guidewire has been obtained in which the guidewire partly extends into the targeted branched passageway of the first bifurcation.

The reference numerals used in Figs. 1-7 are referring to the above-mentioned parts and aspects of the invention, as well as to related parts and aspects, in the following manner.
- 1: catheter
- 2: guidewire
- 3: proximal catheter end
- 4: distal catheter end
- 5: longitudinal direction
- 6: pre-formed straight center line
- 7: one-direction blunt bend line
- 8: straight line
- 9: proximal catheter segment
- 10: distal catheter segment
- 11: tubular element
- 12: guide lumen
- 13: proximal guide port
- 14: distal guide port
- 15: bent-away tubular segment
- 16: straight tubular segment
- 17: sideward deflection direction
- 18: overall deflection angle
- 19: side guide port
- 20: bifurcation of coronary arteries
- 21: main passageway of bifurcation 20
- 22: targeted branched passageway of bifurcation 20
- 23: non-targeted branched passageway of bifurcation 20
- 24: circumferential direction
- 25: distal guidewire end
- 26: catheter hub of catheter 1
- 27: flexible wire of catheter 1
- 28: tubular wall of tubular element 11

Based on the above introductory description, including the brief description of the drawing figures, and based on the above-listed reference signs used in Figs. 1-7, the examples of Figs. 1-7 are readily self-explanatory. The following extra explanations are given.

The catheter 1 of Figs. 1-7 is a catheter according to the invention, as well as according to all of the above-mentioned preferable embodiments of the invention.

In Fig. 1 the reference numeral 26 indicates a catheter hub of the shown catheter 1 according to the invention, the reference numeral 27 indicates a flexible wire of the catheter 1, and the reference numeral 28 indicates a tubular wall of the tubular element 11 of the distal catheter segment 10 of the catheter 1.

In the shown example, the flexible wire 27 is not only extending in the proximal catheter segment 9 but also in the distal catheter segment 10. The part of the flexible wire 27 extending in the proximal catheter segment 9 is connecting the catheter hub 26 to the distal catheter segment 10. The part of the flexible wire 27 extending in the distal catheter segment 10 is connected to the tubular wall 28 of the tubular element 11 of the distal catheter segment 10. In the longitudinal direction 5 of the catheter 1 the flexible wire 27 may have variable flexibility characteristics, as well as variable shape memory characteristics.

In the shown example it is assumed that, purely by way of example, the part of the flexible wire 27 extending in the distal catheter segment 10 is responsible for providing the shape retention memories of the bent-away tubular segment 15 and of the straight tubular segment 16 to return to their pre-formed states, respectively. However, other embodiments of catheters according to the invention are of course possible in which, instead of or in addition thereto, other parts of the bent-away tubular segment 15 and of the straight tubular segment 16 are at least partly responsible for providing said shape retention memories.

In Figs. 1 and 2, as mentioned above, the bent-away tubular segment 15 and the straight tubular segment 16 of the catheter 1 are in their pre-formed states, respectively. In Fig. 1 the reference numeral 5 indicates the longitudinal direction of the catheter 1. In Figs. 1 and 2 the reference numeral 17 indicates the sideward deflection direction of the one-direction blunt bend line 7 relative to the pre-formed straight center line 6 of the straight tubular segment 16. Fig. 1 shows that the one-direction blunt bend line 7 makes the overall deflection angle 18 relative to the pre-formed straight center line 6. In the shown example, as seen in Fig. 1, the pre-formed bent center line of the bent-away tubular segment 15 not only forms the one-direction blunt bend line 7, but also forms the straight line 8 immediately distal from the one-direction blunt bend line 7 and distally ending at the distal catheter end 4.

In Fig. 2 the reference numeral 24 indicates the circumferential direction around the longitudinal direction of the catheter 1. In Fig. 2 this circumferential direction 24 has been depicted around the pre-formed straight center line 6 of the straight tubular segment. From Fig. 2 it can be seen that the side guide port 19, as seen in the circumferential direction 24, is situated on a side of the tubular element 11 facing diametrically away from the sideward deflection direction 17 of the one-direction blunt bend line 7.

The distal guide port 14 and the side guide port 19 of the catheter 1 form two selectively choosable alternative exits through which a distal guidewire end 25 of a guidewire 2 can be guided out of the guide lumen 12, when the guidewire 2 is advanced distally along the longitudinal direction of the catheter 1.

Now, with additional reference to Figs. 3-7, the advantageous effects of the catheter 1 according to the invention are illustrated in connection with the catheter's example use, which was already discussed in the above introductory description.

Figs. 3-7 show the bifurcation 20 of coronary arteries. The bifurcation 20 has the main passageway 21, the targeted branched passageway 22 and the non-targeted branched passageway 23. Figs. 3-7 further show a distal part of the catheter 1 of Figs. 1-2, as well as a distal part of the guidewire 2 having the distal guidewire end 25.

In the situation of Fig. 3 the catheter 1 together with the guidewire 2 is in the above-mentioned first condition, in which the distal guidewire end 25 of the guidewire 2 has been advanced distally into the bent-away tubular segment 15 in such manner that the pre-formed bent center line of the bent-away tubular segment 15 has been deformed a little bit towards a straightened condition thereof. It is seen that in Fig. 3 the distal guidewire end 25 is distal from the side guide port 19, and that, in preparing said first condition, the medical practitioner had deformed the guidewire tip a little bit towards following the pre-formed shape of the bent-away tubular segment 15 a little bit. It is further seen that in Fig. 3 the distal catheter end 4 has reached the bifurcation 20.

Starting-off from the situation of Fig. 3, we now discuss the action to retract the guidewire 2 a little bit relative to the catheter 1 in proximal direction (see arrow A in Fig. 3), to such an extent that the bent-away tubular segment 15 is allowed to at least partly return a bit further towards its pre-formed shape.

This way the situation of Fig. 4 is obtained, which shows the above-mentioned second condition of the catheter 1 and the guidewire 2. In this second condition of Fig. 4, the distal guidewire end 25 is still inside the guide lumen 12, and in the present example the distal guidewire end 25 is still distal from the side guide port 19. Thanks to the last-mentioned retraction of the guidewire 2, the bent-away tubular segment 15 in the second condition of Fig. 4 has been returned more or less into its pre-formed shape. As a result thereof, and as seen in Fig. 4, the bent-away tubular segment 15 now extends into the targeted branched passageway 22 of the first bifurcation 20.

In this lucky scenario (the above-mentioned plan-A scenario), the guidewire 2 may simply be advanced distally out of the distal guide port 14 of the catheter 1 (see arrow B in Fig. 4), so that the targeted branched passageway 22 of the bifurcation 20 can be wired right away.

Fig. 5 shows the situation of Fig. 3 again, however wherein this time the tubular element of the distal catheter segment of the catheter 1 has reached the bifurcation 20 in the shown different orientation relative to the two branches of the bifurcation 20, i.e. in a more or less upside-down orientation relative to that of Fig. 3. Due to this different orientation, the above-mentioned lucky scenario is absent. However, the present invention further allows for the above-mentioned "plan-B" procedure, which is now elucidated as follows.

In the situation of Fig. 5 the catheter 1 together with the guidewire 2 is in the above-mentioned first condition (this time however in said upside-down orientation), in which the distal guidewire end 25 of the guidewire 2 has been advanced distally into the bent-away tubular segment 15 in such manner that the pre-formed bent center line of the bent-away tubular segment 15 has been deformed a little bit towards a straightened condition thereof. It is seen that in Fig. 5 the distal guidewire end 25 is distal from the side guide port 19, and that, in preparing said first condition, the medical practitioner had deformed the guidewire tip a little bit towards following the pre-formed shape of the bent-away tubular segment 15 a little bit.

Starting-off from the situation of Fig. 5, we now discuss the action to retract the guidewire 2 a little bit relative to the catheter 1 in proximal direction (see arrow C in Fig. 5), to such an extent that the bent-away tubular segment 15 is allowed to at least partly return a bit further towards its pre-formed shape.

This way the situation of Fig. 6 is obtained, which shows the above-mentioned second condition of the catheter 1 and the guidewire 2 (this time however in said upside-down orientation). In this second condition of Fig. 6, the distal guidewire end 25 is still inside the guide lumen 12, and in the present example the distal guidewire end 25 is still distal from the side guide port 19. Thanks to the last-mentioned retraction of the guidewire 2, the bent-away tubular segment 15 in the second condition of Fig. 6 has been returned more or less into its pre-formed shape.

However, as a result of said upside-down orientation, and as seen in Fig. 6, the bent-away tubular segment 15 this time extends into the non-targeted branched passageway 23 of the first bifurcation 20. And this time the slight retraction of the guidewire 2 relative to the catheter 1 in proximal direction has led to the situation that the bent-away tubular segment 15, which has at least partly returned a bit further towards its pre-formed shape, in the second condition of the plan-B scenario is pushing on the wall of the non-targeted branched passageway 23. As mentioned, this pushing provides the catheter tip with some extra stability within the blood vessel structure, which is an advantage of the invention since it is helpful for the further plan-B procedure. The further plan-B procedure is elucidated as follows.

Starting-off from the second condition of the plan-B scenario, we now discuss the action to retract the guidewire 2 yet a bit further relative to the catheter 1 in proximal direction (see arrow D in Fig. 6) in such manner that the distal guidewire end 25 of the guidewire 2 comes within the longitudinal range of the side guide port 19. In addition, the guidewire 2 is rotated relative to the catheter 1 over approximately 180 degrees about the longitudinal direction (see arrow E in Fig. 6).

This way the situation of Fig. 7 is obtained, which shows the above-mentioned third condition of the catheter 1 and the guidewire 2. In this third condition, the distal guidewire end 25 is projecting a bit out of the side guide port 19 of the catheter 1, and into the targeted branched passageway 22 of the bifurcation 20.

Starting-off from this third condition of Fig. 7 the guidewire 2 may simply be further advanced distally out of the side guide port 19 (see arrow F in Fig. 7). There is a very good chance that the guidewire will thus be entered right away into the targeted branched passageway 22 of the bifurcation 20, so that the targeted branched passageway 22 of the bifurcation 20 can be wired. A little bit of rotating the guidewire 2 to-and-fro about the longitudinal direction may help to redirect the guidewire 2 into the targeted branched passageway 22.

The invention can be practiced with many various structures, many various materials, many various shapes and many various dimensions of the rapid exchange guide catheter, such as the many various structures, the many various materials, the many various shapes and the many various dimensions that are used in known (rapid exchange) guide catheters.

It is further noted that according to the invention typical dimensions of some parts and aspects of the invention, and applicable practical ranges of such dimensions are as follows. Effective length of the rapid exchange guide catheter (as measured from the distal end of a hub of the catheter) can be in the range between 130 cm and 160 cm, and can typically be 135 cm. Maximum outer diameter of the tubular element of the rapid exchange segment of the rapid exchange guide catheter can be in the range between 0.60 mm and 1.20 mm, and can typically be 0.75 mm, while noting that the word "maximum" in the term "maximum outer diameter" is referring to the maximum value of the outer diameter of the tubular element as considered over the full length of the tubular element. Minimum inner diameter of the tubular element of the rapid exchange segment of the rapid exchange guide catheter can be in the range between 0.36 mm and 0.55 mm, and can typically be 0.45 mm, while noting that the word "minimum" in the term "minimum inner diameter" is referring to the minimum value of the inner diameter of the tubular element as considered over the full length of the tubular element. For example, the following typical combinations of maximum outer diameter and minimum inner diameter of the tubular element are possible: maximum outer diameter 0.75 mm in combination with minimum inner diameter 0.45 mm, maximum outer diameter 1.20 mm in combination with minimum inner diameter 0.55 mm, or maximum outer diameter 0.60 mm in combination with minimum inner diameter 0.36 mm.

## Claims

1. A rapid exchange guide catheter (1) configured for use through coronary arteries (21, 22, 23) for guiding a guidewire (2) through said coronary arteries and for delivering said guidewire into said coronary arteries,
wherein the catheter (1) has a proximal catheter end (3), a distal catheter end (4) and a longitudinal direction (5) extending from the proximal catheter end to the distal catheter end, wherein the catheter (1) comprises a proximal catheter segment (9) and a distal catheter segment (10), which are mutually interconnected in said longitudinal direction, the proximal catheter segment ending at the proximal catheter end (3), the distal catheter segment ending at the distal catheter end (4), the distal catheter segment (10) being a rapid exchange segment of the catheter and comprising a tubular element (11) ending at the distal catheter end (4), wherein the tubular element (11) comprises one and only one guide lumen (12), a proximal guide port (13) at a proximal guide lumen end and a distal guide port (14) at a distal guide lumen end being the distal catheter end (4), wherein the proximal guide port (13), the guide lumen (12) and the distal guide port (14) are each configured for guiding a guidewire (2) therethrough,
wherein the tubular element (11) comprises a bent-away tubular segment (15) ending at the distal catheter end (4) and a straight tubular segment (16) immediately proximal from the bent-away tubular segment (15), the bent-away tubular segment and the straight tubular segment being flexible, and the bent-away tubular segment and the straight tubular segment having pre-formed shapes, respectively, with shape retention memories to return to pre-formed states corresponding to said pre-formed shapes, respectively,
wherein, as seen in said pre-formed states of the straight tubular segment (16) and the bent-away tubular segment (15), the pre-formed shape of the straight tubular segment (16) corresponds to the straight tubular segment having a pre-formed straight center line (6) in said longitudinal direction of the catheter, and the pre-formed shape of the bent-away tubular segment (15) corresponds to the bent-away tubular segment having a pre-formed bent center line (7, 8) in said longitudinal direction of the catheter, said pre-formed bent center line (7, 8) forming a one-direction blunt bend line (7), which in a sideward deflection direction (17) relative to said pre-formed straight center line (6) makes an overall deflection angle (18) having a value in a range between 30 and 90 degrees relative to said pre-formed straight center line (6),
wherein the bent-away tubular segment (15) has a flexibility that allows the pre-formed bent center line (7, 8) to be at least straightened by advancing a guidewire (2) through the bent-away tubular segment (15),
wherein the tubular element (11) further comprises a side guide port (19), said side guide port being configured for guiding a guidewire (2) therethrough,
and wherein the side guide port (19), as seen in said pre-formed states of the straight tubular segment (16) and the bent-away tubular segment (15), and as seen in a circumferential direction (24) around said longitudinal direction (5) of the catheter (1), is situated on a side of the tubular element (11) facing diametrically away from said sideward deflection direction (17) of said one-direction blunt bend line (7),
so that the distal guide port (14) and the side guide port (19) form two selectively choosable alternative exits through which a distal guidewire end (25) of a guidewire (2) can be guided out of the guide lumen (12), when the guidewire is advanced distally along said longitudinal direction of the catheter (1).

2. The rapid exchange guide catheter (1) according to claim 1, wherein said value, relative to said pre-formed straight center line (6), of said overall deflection angle (18) of said one-direction blunt bend line (7) of said pre-formed bent center line (7, 8) of said bent-away tubular segment (15) is in a range between 40 and 85 degrees, more preferably between 50 and 80 degrees, yet more preferably between 60 and 75 degrees, yet more preferably between 68 and 71 degrees, and most preferably is equal to 70 degrees.

3. The rapid exchange guide catheter (1) according to claim 1 or 2, wherein at least part of said side guide port (19) is located in the straight tubular segment (16).

4. The rapid exchange guide catheter (1) according to claim 3, wherein said side guide port (19) as a whole is located in the straight tubular segment (16).

5. The rapid exchange guide catheter (1) according to any one of the preceding claims, wherein said side guide port (19) is elongate in said longitudinal direction (5) of the catheter (1).

6. The rapid exchange guide catheter (1) according to any one of the preceding claims, wherein, as seen in said pre-formed states of the straight tubular segment (16) and the bent-away tubular segment (15), said pre-formed bent center line (7, 8) further forms a straight line (8) immediately distal from said one-direction blunt bend line (7) and distally ending at the distal catheter end (4).
